# EUROPÄISCHE PATENTANMELDUNG

(11) **EP 1 122 326 A1**
(43) Veröffentlichungstag der Anmeldung: **08.08.2001**
(21) Anmeldenummer: 01100724.2
(22) Anmeldetag: 12.01.2001
(51) Int. Cl.: C22C 19/03, C22C 19/07, A61K 6/04

(54) **Nichtedelmetall-Legierung und Verwendung derselben**

(30) Priorität: 14.01.2000 DE 10001547
(71) Anmelder: BEGO Bremer Goldschlägerei Wilh. Herbst GmbH & Co., 28359 Bremen (DE)
(72) Erfinder: Strietzel, Roland Dieter, Dr., 28865 Lilenthal (DE)
(74) Vertreter: Eisenführ, Speiser & Partner

(57) **Zusammenfassung**

Es wird eine Nichtedelmetall-Legierung auf Kobalt-Chrom- bzw. Nickel-Chrom-Basis vorgeschlagen, die 30 - 70 % Nickel und/oder Kobalt, 20 - 35 % Chrom und 3 - 25 %, insbesondere 12 - 15 % Mangan und/oder Eisen enthält. Mit einer solchen Legierung als Gußwerkstoff lassen sich Dentalerzeugnisse wie Kronen, Brücken, Inlays u. dgl. herstellen, welche keramisch verblendet werden können, ohne daß es aufgrund unterschiedlicher Wärmeausdehnungskoeffizienten zu Problemen kommt.

## Beschreibung

Die Erfindung betrifft eine Nichtedelmetall-Legierung auf Kobalt-Chrom- bzw. Nickel-Chrom-Basis sowie deren Verwendung als Gußwerkstoff für Dentalerzeugnisse wie Kronen, Brücken, Inlays u. dgl.

Aus der DE 35 10 331 C1 ist die Verwendung einer Kobalt-Chrom-Gußlegierung für Dentalzwecke, nämlich als Werkstoff für gegossene Gerüste und Platten für herausnehmbare Dentalprothesen bekannt, die neben mindestens 20 (Massen-)% Kobalt 20 - 35 % Chrom, 0,3 - 10,0 % Mangan und 5 - 40 % Eisen sowie 0,1 - 1,0 % Kohlenstoff und ferner Molybdän, Silizium und Stickstoff in geringeren Anteilen enthält. Darüber hinaus waren Nichtedelmetall-Gußlegierungen auf Kobalt-Chrom-Basis für Dentalzwecke schon seit Jahrzehnten bekannt; bekannt war auch, Nickel im teilweisen Austausch gegen Kobalt einzusetzen.

Zweck der in der DE 35 10 331 C1 beschriebenen teilweisen Substitution von Kobalt durch Eisen ist die dadurch bewirkte Kostenersparnis. Die übrigen Legierungsbestandteile haben die Aufgabe, die mit zunehmendem Gehalt an Eisen auf Kosten von dem an Kobalt abnehmende 0,2 %-Dehngrenze zu korrigieren, ohne die Vergießbarkeit und die Biokompatibilität zu gefährden.

Ebenfalls seit Jahrzehnten ist es bekannt, die aus derartigen Legierungen hergestellten dentalen Gußerzeugnisse durch Aufbrennen mit Keramik zu verblenden. Diese Technik hat sich erfolgreich eingeführt und wird in beträchtlichem Umfang eingesetzt. In jüngerer Zeit haben sich jedoch Probleme aus dem Umstand ergeben, daß anderes Keramikmaterial als Verblendwerkstoff für dentale NEM-Gußerzeugnisse eingesetzt wird, welches beim Aufbringen auf Dentalerzeugnisse aus den bekannten Gußlegierungen Schwierigkeiten macht und nicht selten zu Ausschuß führt. Es wurde nun gefunden, daß diese Probleme ihre Ursache in einem höheren Wärmeausdehnungskoeffizienten der anderen Keramiken haben. Liegt der Wärmeausdehnungskoeffizient herkömmlicher Legierungen und Keramiken bei etwa 14 (10⁻⁶ × K⁻¹), findet man bei den neuen Keramiken einen Wert von etwa 16 (10⁻⁶ × K⁻¹). Die Folge ist, daß es aufgrund der unterschiedlichen Materialausdehnungen zu inneren Spannungen, Rissen und gegebenenfalls Abbrüchen kommt.

Der Erfindung liegt die Aufgabe zugrunde, eine Gußlegierung insbesondere für Dentalzwecke anzugeben, die einen im wesentlichen gleichen Wärmeausdehnungskoeffizienten besitzt wie die in jüngerer Zeit eingesetzten Keramiken, so daß es bei deren Verwendung als Gußwerkstoff für dentale Gerüste o.ä. nicht zu jenen Fehlern kommt.

Die Lösung dieser Aufgabe besteht in einer Legierung auf Kobalt-Chrom- bzw. Nickel-Chrom-Basis, die 30 - 70 (Massen-)% Nickel und/oder Kobalt, 20 - 35 % Chrom, 3 - 25 %, insbesondere 12 - 15 % Mangan und/oder Eisen und < 0,1, insbesondere < 0,02 % Kohlenstoff enthält. Zwar kann der Wärmeausdehnungskoeffizient der Legierung auch durch andere Elemente wie Kupfer, Indium, Silber, Zinn oder Zink angehoben werden, jedoch eignen sich auf Grund des Saldos verschiedener Gründe Eisen und Mangan im Besonderen. Zu diesen Gründen gehört zunächst die biologische Verträglichkeit, weil es sich bei Eisen und Mangan um essentielle Elemente handelt. Ferner macht ein höherer Eisen- und/oder Mangan-Gehalt die Legierung weicher und deshalb besser verarbeitbar; insbesondere eine gute Gießbarkeit und eine gute Laser-Schweißbarkeit zeichnen die erfindungsgemäße Legierung aus.

Hinzu kommt die hohe Korrosionsbeständigkeit der Legierung, welche ebenso wie die schon angesprochenen mechanischen Eigenschaften durch einen Zusatz von 4 - 15 % Molybdän und/oder 1 - 15 % Wolfram und/oder Niob zusätzlich erhöht werden können. Auch Zusätze von 0 - 5 % Aluminium, Lanthanoiden (seltene Erden), insbesondere von Cer und von Titan und/oder Zirkonium sind ebenso wie ein Aufsticken geeignet, die mechanischen Eigenschaften der Legierung weiter zu verbessern. Eine Verbesserung der Schmelzeigenschaften der Legierung kann durch Zusätze im Bereich von 0 - 6 % Silizium, Bor und/oder Germanium erzielt werden; auch eignen sich diese Zusätze als Sauerstofffänger und machen die Legierung insbesondere für Lote interessant. Schließlich ist nicht ausgeschlossen, der Legierung auch 0 - 10% an Edelmetallen, also namentlich Silber, Gold und/oder Metallen aus der Platingruppe hinzuzufügen.

Eine als Gußwerkstoff für Dentalerzeugnisse wie Kronen, Brücken, Inlays u. dgl. geeignete erfindungsgemäße NEM-Legierung kann etwa wie folgt zusammengesetzt sein:

### Beispiel 1

| | |
|---|---|
| Kobalt | 50,4 % |
| Chrom | 29,0 % |
| Mangan | 14,0 % |
| Molybdän | 5,0 % |
| Silizium | 1,3 % |
| Stickstoff | 0,3 % |

### Beispiel 2

| | |
|---|---|
| Kobalt | 49,0 % |
| Chrom | 29,0 % |
| Eisen | 15,4 % |
| Molybdän | 5,0 % |
| Silizium | 1,3 % |
| Stickstoff | 0,3 % |

## Patentansprüche

1. Nichtedelmetall-Legierung auf Kobalt-Chrom- bzw. Nickel-Chrom-Basis enthaltend
30 - 70 % Nickel und/oder Kobalt
20 - 35 % Chrom
3 - 25 %, insbesondere 12 - 15 % Mangan und/oder Eisen.

2. Nichtedelmetall-Legierung nach Anspruch 1,
gekennzeichnet durch einen Zusatz von 4 - 15 % Molybdän.

3. Nichtedelmetall-Legierung nach Anspruch 1 oder 2, ferner enthaltend, allein oder in Kombination
4 - 15 % Molybdän
1 - 15 % Wolfram und/oder Niob
0 - 6 % Silizium, Bor und/oder Germanium
0 - 5 % Aluminium, Titan, Zirkonium und/oder Lanthanoide, insbesondere Cer < 0,1, insbesondere < 0,02 % Kohlenstoff
0 - 0,4 % Stickstoff.

4. Nichtedelmetall-Legierung nach einem der Ansprüche 1 bis 3,
dadurch gekennzeichnet, daß der Mangan- und/oder Eisenanteil ganz oder teilweise - allein oder in Kombination - durch Kupfer, Indium, Silber, Zinn, Zink ersetzt wird.

5. Verwendung einer Nichtedelmetall-Legierung gemäß einem der vorhergehenden Ansprüche als Gußwerkstoff für Dentalerzeugnisse wie Kronen, Brücken, Inlays u. dgl.
